# EUROPEAN PATENT SPECIFICATION

(11) **EP 1 749 489 B1**
(45) Date of publication and mention of the grant of the patent: **17.11.2010**
(21) Application number: 05016901.0
(22) Date of filing: 03.08.2005
(51) Int. Cl.: A61B 17/70

(54) **Bone anchoring device**
Knochenverankerungsvorrichtung
Dispositif d'ancrage osseux

(43) Date of publication of application: 07.02.2007
(73) Proprietor: BIEDERMANN MOTECH GmbH, 78054 VS-Schwenningen (DE)
(72) Inventor: Biedermann, Lutz, 78048 VS-Villingen (DE); Matthis, Wilfried, 79367 Weisweil (DE)
(74) Representative: Hofer, Dorothea

(56) References cited:
- WO-A-03/043511
- WO-A-03/096915
- WO-A-2005/122965
- DE-A1- 4 330 837
- US-A- 5 899 904
- US-A1- 2005 038 433
- US-A1- 2005 059 972

## Description

The invention relates to a bone anchoring device comprising an anchoring element which has a shank to be anchored in a bone and a head, a receiving portion to receive said head and an element which exerts pressure on said head, wherein said head and said receiving portion are connected such that a longitudinal axis of the receiving portion and the shank axis have a fixed angle relative to each other and wherein the receiving portion and the head are rotatable relative to each other.

A bone anchoring device comprising a shank to be anchored in a bone and a receiving portion to connect the shank with a rod, wherein the receiving portion and the shank are formed as a single piece is known, for example, from US 5,005,562 or from DE 101 57 969 C1. At the time of screwing in such a so-called monoaxial bone screw the receiving portion is aligned to receive the rod by rotating the shank within its fixation in the bone. The depth into which the shank can be screwed into the bone, depends on the required orientation of the receiving portion relative to the rod. Therefore, the possibility of fine-adjusting the orientation of the receiving portion relative to the rod depends on the thread pitch of the threaded shank. Usually monoaxial bone screws can not be screwed-in to be fully seated within the anatomical dimensions.

A polyaxial bone anchoring device, wherein the shank and the head are separate parts, is known from US 6,835,196 B2. This bone anchoring device comprises a shank to be anchored in the bone and a head which has an exterior surface with a spherical segment-shaped portion, a receiving portion to receive said head and an element which exerts pressure on said head. The shank is received in a bore in the head which has a spring-yielding edge to clamp said end portion of the shank when pressure is exerted on the head. Since the head has an exterior surface with a spherical segment-shaped portion, the head can be pivoted in the receiving portion as long as a pressure is not exerted onto the head. With this bone anchoring device it is possible to first screw in the shank, adjust the length of the shank, if necessary, and then connect it with the receiving portion containing the head.

For certain clinical applications monoaxial bone screws are more appropriate than polyaxial bone screws. However, the known monoaxial bone screws have the disadvantage that there is only a small possibility of alignment of the receiving portion relative to the rod and yet be fully seated in the bone so as to be flush with the bone surface.

WO03/043511 describes a device for joining a longitudinal support with a bone fixation means. The device comprises a joining piece and a bone fixation means with a cylindrical head which is accommodated in the joining piece. A longitudinal support is also accommodated in the joining piece and a cap is provided for pressing onto the longitudinal support which presses onto the head of the fixation means.

US 2005/0038433 A1 discloses a clamp which clamps a rod by means of a collet. A nut is used pressing together the parts of the clamp which press onto the collet.

US 5,899,904 discloses a rod, screw, rod-securing member and a staple assembly for use in conjunction with anterior or lateral spinal rod implant apparatus. It includes a vertebral body screw and a rod-coupling member which are loosely coupled by virtue of the head of the screw being held within a contractible volume in the base of the rod-coupling member. A top locking nut causes the rod to compress against the staple and to lock the head thereby.

DE 43 30 837 discloses a pedicle screw with a receiving member which can be placed onto shaft part and can be locked in defined rotational positions.

WO 2005/122965 A2 is a prior art pursuant to Art. 54(3) EPC and discloses a bone anchoring device comprising an anchoring element comprising a shank to be anchored in a bone or a vertebra and having a shank axis, and a head; a receiving portion comprising a first end and a second end opposite to the first end, a longitudinal axis passing through the two ends, a bore coaxial with the longitudinal axis, said bore having a smaller diameter adjacent to the second end to form a first region for receiving a section of said head, and a U-shaped recess starting at the first end and extending in the direction of the second end to receive a rod, by the U-shaped recess, two free legs are formed ending towards the first end; an element screwable between the legs, which exerts pressure on said head to lock said head in the receiving portion; wherein the receiving portion and the anchoring element are connected such that the longitudinal axis of the receiving portion and the shank axis have a fixed angle relative to each other and wherein the receiving portion and the shank are rotatable with respect to each other around the longitudinal axis when the pressure is not exerted on the head and are locked in the rotational position when the pressure is exerted on the head, wherein said shank and said head are separate parts.

It is an object of the invention to provide a bone anchoring device of the monoaxial type with an improved possibility of alignment of the receiving portion relative to the rod and yet be fully seated in the bone so as to be flush with the bone surface. Further, the bone anchoring device shall have a simple construction and shall be easy to handle.

The object is solved by a bone anchoring device according to claim 1. Further developments are given in the dependent claims.

The bone anchoring device according to the invention has the advantage that the receiving portion is easily adjustable relative to the rod in a range of 360° around the shaft axis independently from the depth to which the shank is screwed into the bone. The alignment is independent from the thread pitch of a bone thread provided at the shank. Therefore, the shank can be screwed into the bone to be fully seated therein so as to be flush with the bone surface.

A further advantage is that a bone thread with a greater thread pitch compared to the conventional monoaxial bone screws or a multiple-thread can be used.

In addition, it is possible to use the receiving portion and the shank of known polyaxial bone anchoring devices, such as disclosed in US 6,835,196 B2 by simply combining it with the head according to the invention to provide for a monoaxial bone screw.

Further features and advantages of the invention will become apparent from the description of embodiments in conjunction with the accompanying drawings.
- Fig. 1: shows a cross-sectional view of the bone anchoring device according to a first embodiment of the invention.
- Fig. 2: shows an exploded view of the bone anchoring device according to Fig. 1.
- Fig. 3: shows a perspective view of the head of the bone anchoring element, which is part of the bone anchoring device according to Fig. 1.
- Fig. 4: shows a cross-sectional view of the bone anchoring device according to a second embodiment of the invention.
- Fig. 5: shows an exploded view of the bone anchoring device according to Fig. 4.
- Fig. 6: shows a perspective view of the head of the bone anchoring element which is part of the bone anchoring device according to Fig. 4.
- Fig. 7: shows a cross-sectional view of the bone anchoring device according to a third embodiment.

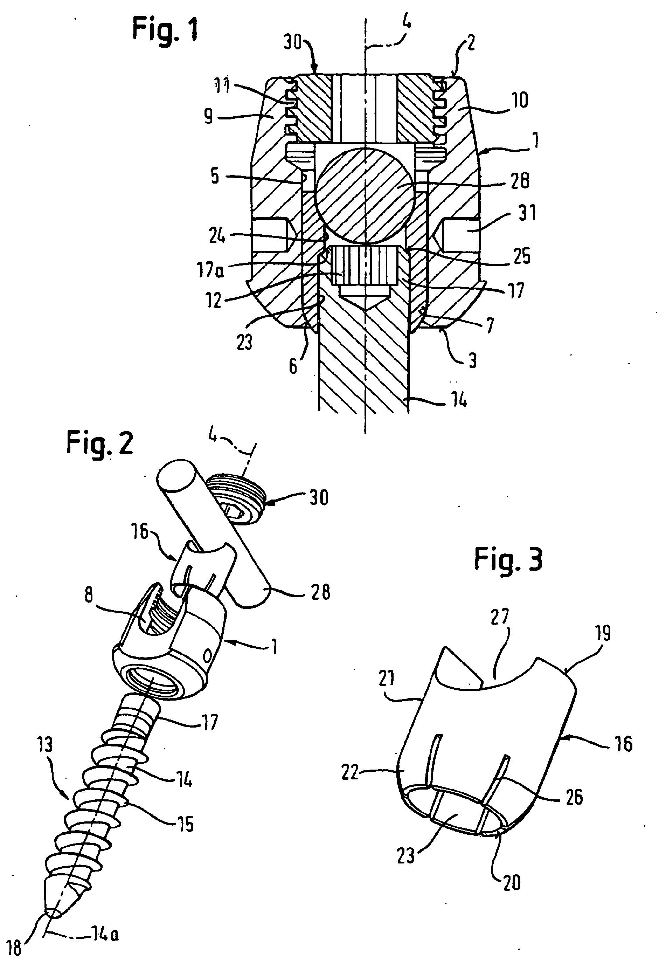

As shown in Figs. 1 to 3, the bone anchoring device comprises a receiving portion 1, which is substantially cylindrical and has a first end 2 and a second end 3 opposite to the first end. The two ends are perpendicular to a longitudinal axis 4. Coaxially with the longitudinal axis 4 a bore 5 is provided which extends from the first end 2 to a predetermined distance from the second end 3. At the second end 3 an opening 6 is provided, the diameter of which is smaller than the diameter of the bore 5. The coaxial bore 5 tapers towards the opening 6 in form of a spherically-shaped section 7.

The receiving portion 1 further has a U-shaped recess 8 which starts at the first end 2 and extends in the direction of the second end 3 to a predetermined distance from the second end 3. By means of the U-shaped recess, two free legs 9, 10 are formed ending towards the first end 2. Adjacent to the first end 2 the receiving portion 1 comprises an internal thread 11 at the legs 9, 10.

The bone anchoring element 13 comprises a shank 14 with a bone thread 15 and a head 16, which is formed as a separate part and which is connected to the shank in the assembled state shown in Fig. 1. The shank 14 has a shank axis 14a and comprises a cylindrical end portion 17 to be received in the head 16 and a tip 18 on the opposite end. The cylindrical end portion is provided with a chamfer 17a to facilitate insertion into the head 16. At its end surface, the end portion comprises a recess 12 for engagement with a screwing-in tool.

The head 16 is substantially cylindrically shaped and comprises a first end 19 and a second end 20 opposite to the first end. As can be seen in particular in Fig. 3 the head 16 has a first portion 21 adjacent to the first end 19, having a cylindrical outer surface. The diameter of the first portion 21 is just as large that the head 16 can slide in the bore 5. Adjacent to the first portion 21, there is a second portion 22, in which the outer surface of the head 16 tapers towards the second end 20. In this embodiment, the second portion 22 has a spherical segment-shaped outer surface. The radius of the spherical segment corresponds to the radius of the spherical section 7 of the receiving part such that the head 16 rests with its second portion 22 or a part thereof in the spherical portion of the receiving part 1. As can be seen in particular in Fig. 1, the head 16 further comprises a first coaxial bore 23 extending from the second end 20 in direction to the first end 19. The diameter of the first coaxial bore 23 is just as large that the end portion 17 of the shank can be inserted into the bore from the second end 20. A second coaxial bore 24 is provided which extends from the end of the first bore 23 to the first end 19. The diameter of the second coaxial bore 24 is smaller than the diameter of the first coaxial bore 23 such that at the transition of the first bore 23 to the second bore 24 a circular abutment surface 25 is provided which forms a stop for the end portion 17 of the shank when the end portion 17 is inserted into the first coaxial bore 23 of the head 16.

In the second portion 22 of the head a plurality of slits 26 are provided which are open towards the second end 20 and extend through the second portion 22 to a predetermined distance from the second end 20. In the embodiment shown in Fig. 3 six slits 26 are provided which are spaced equidistantly in a circumferential direction. However, fewer or more slits can be provided. At least one slit is required to render the second portion elastic to clamp the end portion 17 of the shank. The second bore 24 allows for accessing the recess 12 provided at the end surface of the end portion 17 of the shank for screwing-in the shank into the bone with a screwing-in tool.

The head 16 further comprises a cylindrical segment-shaped recess 27 starting at the first end 19 and extending in the direction to the second end 20. The cylinder axis of the recess 27 is perpendicular to the longitudinal axis 4. The radius of the recess 27 corresponds to the radius of a rod 28 to be received in the recess 27. The size of the recess 27 is such that when the rod 28 is inserted, it protrudes over the first end 19.

The bone anchoring device further comprises an inner screw 30, which can be screwed-in between the legs 9, 10 to fix the rod 28 and to exert a pressure via the rod 28 on the head 16. The internal thread 11 and the cooperating thread of the inner screw 30 can have any known thread shape. A flat thread or a negative angle thread however, has the advantage that a splaying of the legs 9, 10 does not occur which makes it unnecessary to use an outer nut or a ring.

The length of the head 16 is such that in an assembled state shown in Fig. 1 the head 16 encompasses the end portion 17 of the shank with the spherical segment-shaped second portion 22 and a part of the cylindrical portion 21.

The parts of the bone anchoring device described above are preferably made of a body compatible material, such as for example, titanium.

In a first mode of operation, a shank 14 of suitable length and with a desired bone thread 15 is selected and connected with its end portion 17 to the head 16 by introducing it from the second end 20 of the head 16 into the first bore 23 until the end surface abuts to the abutment surface 25. The introduction of the end portion 17 into the head 16 from the second side 20 is facilitated by the chamfer 17a. The end portion 17 is provisionally held in the head 16 by means of friction.

Then, the bone anchoring element 13 consisting of the shank 14 with the mounted head 16 is introduced into the receiving portion 1 from the first end 2 with the threaded shank 14 being guided through the opening 6 of the receiving portion until the head 16 rests against the edge of the opening 6. The head and the shank are rotated such that the recess 27 of the head is aligned with the U-shaped recess 8 of the receiving portion. In such a preassembled state which can be loosely held by crimp bores 31 shown in Fig. 1, the shank is screwed into the bone with a screwing-in tool which engages the recess 12 in the end portion 17 through the second bore 24 in the head. The shank 14 can be fully screwed into the pre-drilled core hole without the necessity of alignment of the receiving portion during the screwing-in procedure.

When the shank is fully screwed-in, the receiving portion together with the head can be rotated around the longitudinal axis 4 by 360° to align it with respect to the rod 28 to be inserted. After aligning the receiving portion 1 the rod 28 is inserted until it rests in the cylindrical recess 27 of the head. Then the inner screw 30 is inserted and tightened until it presses onto the rod 28 which itself presses the head 16 against the spherical portion 7 of the receiving portion. By pressing the head 16 against the spherical section 7 of the receiving portion the spherical portion 22 of the head is compressed due to the slits 26 and firmly clamps the shank. At the same time the head is pressed against the spherical portion 7 and locked relative to the receiving portion 1 in its rotational position.

In a second mode of operation, the threaded shank 14 is first screwed into the bone or into a vertebra. Then, the receiving portion 1 together with the loosely preassembled head 16 is pressed onto the end portion 17 of the shank projecting out of the bone. Thereafter, the receiving portion 1 is aligned such as to be able to receive the rod 28. Then the inner screw 30 is screwed-in to fix to the head and the rod.

With the bone anchoring device it is possible to use a shank 14 with a bone thread 15 having a larger thread pitch than conventional monoaxial bone screws. Also the use of multiple-threads for the bone thread is possible. Since the receiving portion can be rotated by 360° around the shaft axis, the shaft can be screwed-in to the maximum depth in the core hole.

It is further possible to use the receiving portion of conventional polyaxial bone screws together with the head and shank of this embodiment.

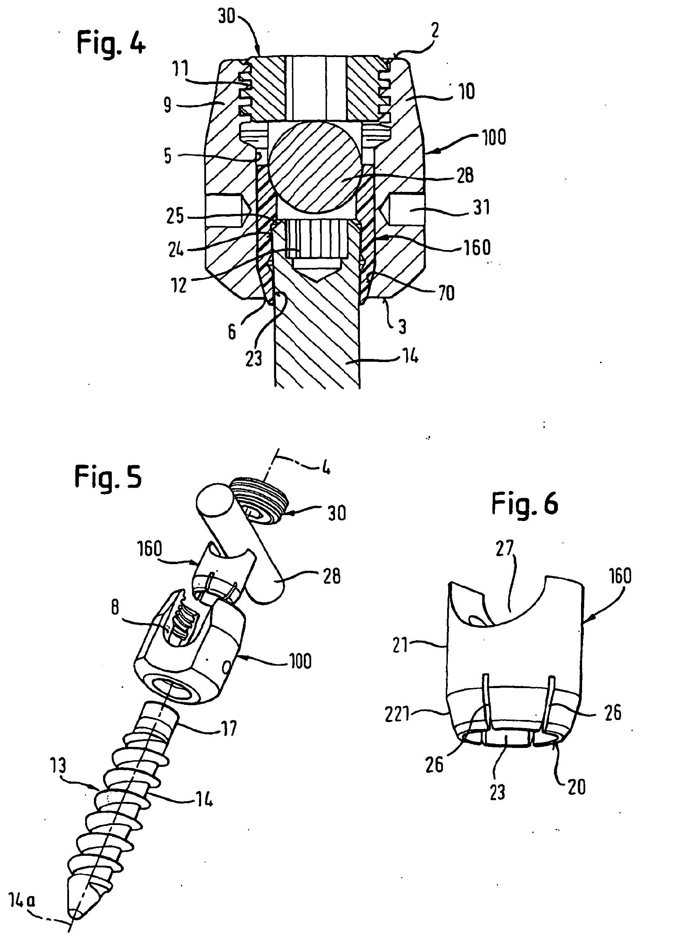

In the second embodiment shown in Figs. 4 to 6, parts which are identical to the first embodiment are provided with the same reference signs as in the first embodiment. The second embodiment differs from the first embodiment in the shape of the section of the receiving portion against which the head rests and the shape of the head. The receiving portion 100 of the second embodiment has instead of the spherical segment-shaped section 7 a conically tapering section 70 adjacent to the second end 3. Correspondingly the head 160 has a second portion 221 with an outer surface which conically tapers towards the second end 20. The cone angle of the second portion 221 of the head corresponds to the cone angle of the portion 70 of the receiving portion. The cone angle is selected such that in a preassembled state the receiving portion 100 is still rotatable relative to the head 160.

The modes of operation are identical to the first embodiment. Description thereof shall not be repeated.

The bone anchoring device of the second embodiment has the advantage that the head having the conical outer surface is easier to manufacture.

Modifications of the embodiments are possible. The diameter of the threaded portion of the shank 14 can be smaller or larger than the diameter of the end portion 17. The end portion 17 can have a conical shape such that the diameter increases towards the free end. The corresponding first bore 23 of the head can have a corresponding conical shape. In this case the diameter of the first bore 23 is slightly smaller than the diameter of the end portion. The slits 26 allow an easy opening of the bore 23 and after inserting the end portion of the shank the end portion is slightly clamped. The cone angle can be selected such that a self locking occurs when the shank is inserted into the head. The head can have one or more slits which are open towards the first end 19.

In a further modification, the head is formed of a material providing elasticity, for example a suitable plastic material. In this case, the slits may be omitted.

The receiving portion can be modified, too. The radius of the spherical section 7 of the receiving portion of the first embodiment can be larger than the radius of the head, as long as the opening is small enough that the head cannot fall out. It is even possible to have a shape which is not spherical but rounded or otherwise tapered towards the opening.

The head 16 can have a U-shaped recess instead of the cylindrical recess 27 in such a way that by the U-shaped recess two free legs are formed. The dimensions of the U-shaped recess are such that the legs project above the rod when the rod is inserted. In this case it is possible to separately fix the head 16 via the inner screw without fixing the rod. To fix the rod, a separate inner screw is necessary.

The head also can have a flat free end surface.

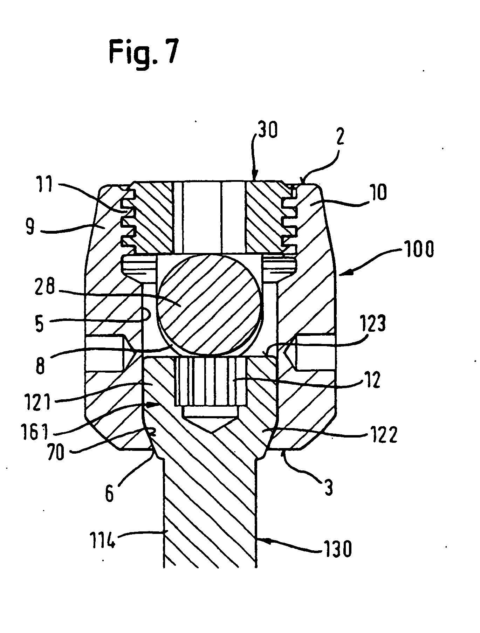

In the embodiments described the shank and the head are separate parts. However, it is also conceivable that the shank and the head are formed as a single piece bone anchoring element. Fig. 7 shows a third embodiment which comprises a bone anchoring element with a shank and a head formed as a single piece.

Parts of this embodiment which are identical to the second or first embodiment have the same reference numerals as those of the second or first element. The receiving portion 100 is shaped as the receving portion of the second embodiment shown in Fig. 4 which has the conically tapering region 70 adjacent to the second end 3. The bone anchoring element 130 comprises a shank 114 having a bone thread (not shown) and a head 161 formed as a single piece. The head 161 has a first portion 121 having a cylindrical outer surface and an adjacent second portion 122 having a conical outer surface tapering towards the shank. The diameter of the first portion is such that the head can slide in the bore 5 of the receiving portion 100. The cone angle of the conical portion 122 corresponds to that of the conical portion 70 of the receiving portion. The free end surface 123 of the head is flat and has a recess 12 for engagement with a screwing-in tool. The flat surface ensures that it is possible to align the receiving portion.

In use the bone anchoring element 130 is first inserted into the receiving portion 100, then it is screwed into the bone.

When it is fully seated in the bone, the receiving portion is still rotatable with respect to the head and thus can be aligned to receive the rod. After inserting the rod 28 the inner screw 30 is tightened and exerts pressure on the rod 28 which exerts pressure on the head 161. The head is pressed against the opening 6 of the receiving portion or against the conical section 70 and is locked in its rotational position.

Modifications are possible. The conical section of the receiving portion can have another shape. The head can have, for example, a sperical section instead of the conical section.

## Claims

1. Bone anchoring device comprising
an anchoring element (13) comprising a shank (14) to be anchored in a bone or a vertebra and having a shank axis (14a), and a head (16; 160);
a receiving portion (1; 100) comprising a first end (2) and a second end (3) opposite to the first end (2), a longitudinal axis (4) passing through the two ends, a bore (5) coaxial with the longitudinal axis (4), said bore (5) having a smaller diameter adjacent to the second end (3) to form a first region (7; 70) for receiving a section (22; 221) of said head, and a U-shaped recess (8) starting at the first end (2) and extending in the direction of the second end (3) to receive a rod (28), by the U-shaped recess (8), two free legs (9, 10) are formed ending towards the first end (2);
an element (30) screwable between the legs (9, 10), which exerts pressure on said head (16; 160) to lock said head in the receiving portion;
wherein the receiving portion (1) and the anchoring element (13) are connected such that the longitudinal axis (4) of the receiving portion (1) and the shank axis (14a) have a fixed angle relative to each other and wherein the receiving portion (1) and the shank (14) are rotatable with respect to each other around the longitudinal axis (4) when the pressure is not exerted on the head and are locked in the rotational position when the pressure is exerted on the head; wherein
said shank (14) and said head (16; 160) are separate parts,
said head (16) has a portion (22, 221) which elastically clamps said shaft (14) when pressure is exerted on the head (16; 160) ; and
said head (16; 160) has a first end (19) and a second end (20) and a cylindrical recess (27) extending from the first end in the direction of the second end to receive a rod (28).

2. The bone anchoring element of claim 1, wherein said head (16; 160) has an exterior surface with a cylindrical portion (21).

3. The bone anchoring element of one of claims 1 to 2wherein the head (16; 160) has a hollow interior portion (23) to receive the shank.

4. The bone anchoring element of one of claims 1 to 3, wherein the head has a first end (19) and a second end (20) and a portion (22; 221) to clamp the shank tapers towards the second end (20).

5. The bone anchoring element of claim 4, wherein the portion (22) to clamp the shank is spherically-shaped.

6. The bone anchoring element of claim 4, wherein the portion (221) to clamp the shank is conically-shaped.

7. The bone anchoring element of one of claims 4 to 6, wherein the portion (22; 221) to clamp the head comprises at least one slit (26).

8. The bone anchoring element of one of claims 1 to 7, wherein said first region (7; 70) of the receiving portion (1) for receiving the head (16; 160) tapers towards the second end (3)

9. The bone anchoring element of one of claims 1 to 8, wherein the longitudinal axis (4) of the receiving portion (1; 100) and the shank axis (14a) are coaxial.

## Patentansprüche

1. Knochenverankerungsvorrichtung mit
einem Verankerungselement (13) mit einem Schaft (14), der in einem Knochen oder einem Wirbel zu verankern ist und eine Schaftachse (14a) aufweist, und einem Kopf (16; 160);
einem Aufnahmeabschnitt (1; 100) mit einem ersten Ende (2) und einem zweiten Ende (3) gegenüber dem ersten Ende (2), einer Längsachse (4), die durch die beiden Enden hindurch tritt, einer Bohrung (5), die koaxial zu der Längsachse (4) ist, wobei die Bohrung (5) einen kleineren Durchmesser angrenzend an dem zweiten Ende (3) hat, um einen ersten Bereich (7; 70) zum Aufnehmen eines Bereichs (22; 221) des Kopfs zu bilden, und einer U-förmigen Ausnehmung (8), die an dem ersten Ende (2) startet und sich in der Richtung zum zweiten Ende (3) erstreckt, um einen Stab (28) aufzunehmen, wobei durch die U-förmige Ausnehmung (8) zwei freie Schenkel (9, 10) gebildet sind, die zu dem ersten Ende (2) enden;
einem Element (30), das zwischen die Schenkel (9, 10) schraubbar ist, das einen Druck auf den Kopf (16; 160) ausübt, um den Kopf in dem Aufnahmeabschnitt zu verriegeln;
wobei der Aufnahmeabschnitt (1) und das Verankerungselement (13) derart verbunden sind, dass die Längsachse (4) des Aufnahmeabschnitts (1) und die Schaftachse (14a) einen festen Winkel relativ zueinander haben, und wobei der Aufnahmeabschnitt (1) und der Schaft (14) um die Längsachse (4) zueinander drehbar sind, wenn der Druck nicht auf den Kopf ausgeübt wird, und wobei sie in der Drehposition verriegelt sind, wenn der Druck auf den Kopf ausgeübt wird; wobei
der Schaft (14) und der Kopf (16; 160) separate Teile sind,
wobei der Kopf (16) einen Abschnitt (22, 221) hat, der den Schaft (14) elastisch klemmt, wenn der Druck auf den Kopf (16; 160) ausgeübt wird; und
wobei der Kopf (16; 160) ein erstes Ende (19) und ein zweites Ende (20) sowie eine zylindrische Ausnehmung (27) hat, die sich von dem ersten Ende in der Richtung zu dem zweiten Ende erstreckt, um einen Stab (28) aufzunehmen.

2. Knochenverankerungselement gemäß Anspruch 1, wobei der Kopf (16; 160) eine Außenfläche mit einem zylindrischen Abschnitt (21) hat.

3. Knochenverankerungselement gemäß einem der Ansprüche 1 bis 2, wobei der Kopf (16; 160) einen hohlen inneren Abschnitt (23) zum Aufnehmen des Schafts hat.

4. Knochenverankerungselement gemäß einem der Ansprüche 1 bis 3, wobei der Kopf ein erstes Ende (19) und ein zweites Ende (20) sowie einen Abschnitt (22; 221) zum Klemmen des Schafts hat, der zu dem zweiten Ende (20) abgeschrägt ist.

5. Knochenverankerungselement gemäß Anspruch 4, wobei der Abschnitt (22) zum Klemmen des Schafts kugelförmig ist.

6. Knochenverankerungselement gemäß Anspruch 4, wobei der Abschnitt (221) zum Klemmen des Schafts konisch ist.

7. Knochenverankerungselement gemäß einem der Ansprüche 4 bis 6, wobei der Abschnitt (22; 221) zum Klemmen des Kopfs zumindest einen Schlitz (26) aufweist.

8. Knochenverankerungselement gemäß einem der Ansprüche 1 bis 7, wobei der erste Bereich (7; 70) des Aufnahmeabschnitts (1) zum Aufnehmen des Kopfs (16; 160) zu dem zweiten Ende (3) abgeschrägt ist.

9. Knochenverankerungselement gemäß einem der Ansprüche 1 bis 8, wobei die Längsachse (4) des Aufnahmeabschnitts (1; 100) und die Schaftachse (14a) koaxial sind.

## Revendications

1. Dispositif d'ancrage osseux comprenant :
un élément d'ancrage (13) comprenant une bande de jambe (14) à ancrer dans un os ou une vertèbre et ayant un axe (14a) de la bande de jambe, et une tête (16; 160);
une partie de réception (1; 100) comprenant une première extrémité (2) et une deuxième extrémité (3) opposée à la première extrémité (2), un axe longitudinal (4) passant à travers les deux extrémités, un alésage (5) coaxial avec l'axe longitudinal (4), ledit alésage (5) ayant un diamètre plus petit adjacent à la deuxième extrémité (3) pour former une première région (7; 70) destinée à recevoir un tronçon (22; 221) de ladite tête, et un évidement en forme de U (8) commençant au niveau de la première extrémité (2) et s'étendant en direction de la deuxième extrémité (3) pour recevoir une tige (28), au niveau de l'évidement en forme de U (8), deux jambes libres (9, 10) sont formées se terminant vers la première extrémité (2);
un élément (30) que l'on peut visser entre les jambes (9, 10), qui exerce une pression sur ladite tête (16; 160) pour verrouiller ladite tête dans la partie de réception;
dans lequel la partie de réception (1) et l'élément d'ancrage (13) sont connectés de sorte que l'axe longitudinal (4) de la partie de réception (1) et l'axe (14a) de la bande de jambe présentent un angle fixe l'un par rapport à l'autre et dans lequel la partie de réception (1) et la bande de jambe (14) peuvent tourner l'une par rapport à l'autre autour de l'axe longitudinal (4) lorsque la pression n'est pas exercée sur la tête et sont verrouillées dans la position de rotation lorsque la pression est exercée sur la tête; dans lequel
ladite bande de jambe (14) et ladite tête (16; 160) sont des pièces séparées;
ladite tête (16) a une partie (22, 221) qui serre de manière élastique ledit arbre (14) lorsqu'on exerce une pression sur la tête (16; 160); et
ladite tête (16; 160) a une première extrémité (19) et une deuxième extrémité (20) et un évidement cylindrique (27) s'étendant de la première extrémité en direction de la deuxième extrémité pour recevoir une tige (28).

2. Elément d'ancrage osseux de la revendication 1, dans lequel ladite tête (16; 160) a une surface extérieure avec une partie cylindrique (21).

3. Elément d'ancrage osseux de l'une des revendications 1 à 2, dans lequel la tête (16; 160) a une partie intérieure creuse (23) destinée à recevoir la bande de jambe.

4. Elément d'ancrage osseux de l'une des revendications 1 à 3, dans lequel la tête a une première extrémité (19) et une deuxième extrémité (20) et une partie (22; 221) pour serrer la bande de jambe s'effile vers la deuxième extrémité (20).

5. Elément d'ancrage osseux de la revendication 4, dans lequel la partie (22) destinée à serrer la bande de jambe est de forme sphérique.

6. Elément d'ancrage osseux de la revendication 4, dans lequel la partie (221) destinée à serrer la bande de jambe est de forme conique.

7. Elément d'ancrage osseux de l'une des revendications 4 à 6, dans lequel la partie (22; 221) destinée à serrer la tête comprend au moins une fente (26).

8. Elément d'ancrage osseux de l'une des revendications 1 à 7, dans lequel ladite première région (7; 70) de la partie de réception (1) destinée à recevoir la tête (16; 160) s'effile vers la deuxième extrémité (3).

9. Elément d'ancrage osseux de l'une des revendications 1 à 8, dans lequel l'axe longitudinal (4) de la partie de réception (1; 100) et l'axe (14a) de la bande de jambe sont coaxiaux.
